Europäisches Patentamt

(19) European Patent Office

Office européen des brevets

(11) Numéro de publication: **0 238 983**
**B1**

(12) **FASCICULE DE BREVET EUROPEEN**

(45) Date de publication du fascicule du brevet:
26.09.90

(21) Numéro de dépôt: **87103906.1**

(22) Date de dépôt: **17.03.87**

(51) Int. Cl.⁵: **A61L 9/03**

(54) Dispositif automatique pour la diffusion de vapeurs assainissantes.

(30) Priorité: **27.03.86 CH 1256/86**

(43) Date de publication de la demande:
**30.09.87 Bulletin 87/40**

(45) Mention de la délivrance du brevet:
**26.09.90 Bulletin 90/39**

(84) Etats contractants désignés:
**CH DE ES FR GB IT LI**

(56) Documents cités:
**EP-A- 0 104 758**
**EP-A- 0 123 746**

(73) Titulaire: **FIRMENICH SA, 1, route des Jeunes Case Postale 239, CH-1211 Genève 8(CH)**

(72) Inventeur: **Pougalan, Marc Francis, 9, allée Chimène, F-77420 Noisiel(FR)**

(74) Mandataire: **Salvadori, Giuseppe, Dr., c/o Firmenich S.A. Case Postale 239, CH-1211 Genève 8(CH)**

**Description**

La présente invention a trait à un dispositif automatique pour la diffusion de vapeurs assainissantes dans l'atmosphère environnante et, plus particulièrement, elle concerne un dispositif permettant l'activation automatique et répétitive de l'émission de vapeurs assainissantes pendant un laps de temps déterminé.

Le marché des désodorisants d'air ambiant a acquis une importance grandissante au cours de la dernière décennie et plusieurs techniques ont été proposées afin de réaliser la fabrication d'articles destinés à masquer ou neutraliser les mauvaises odeurs de locaux d'habitation ou publics ou d'enceintes fermées.

Parmi les techniques proposées, la plupart font appel à l'utilisation de parfums comme principes actifs uniques et leur diffusion est réalisée à l'aide d'articles divers tels des flacons "sprays", aérosols ou mécaniques, ou des supports solides parfumés de nature variée. Parmi ces derniers figurent des dispositifs comportant en tant qu'élément actif une pastille ou un disque en carton ou en matière fibreuse végétale ou polymérique imbibée de substance assainissante active, parfumante, désodorisante, insecticide, insectifuge ou bactéricide.

L'art antérieur fait également état d'articles comportant des dispositifs d'activation divers dont la fonction est d'accélérer la diffusion des vapeurs d'agent assainissant dans l'atmosphère. A cet effet, il a été suggéré le recours à une source de chaleur ou à une ventilation forcée.

Le brevet EUA nº 4,346,059 octroyé le 24 août 1982 à Donald Spector en décrit un exemple, la source de chaleur étant fournie par une ampoule électrique située à l'intérieur d'une chambre fermée comportant dans sa partie supérieure un tampon poreux absorbant imbibé d'un agent parfumant. Dans l'article décrit, l'activation et la diffusion des vapeurs parfumantes qui s'ensuivent ont lieu par l'allumage de l'ampoule électrique, ce qui crée un dégagement de chaleur et une différence de pression entre l'air ainsi réchauffé et l'atmosphère au-dessus du tampon imbibé entraînant une circulation forcée de l'air chaud à travers le tampon. D'autres dispositifs comportant un système d'activation électrique ont été proposés par le passé. Par exemple, le brevet EUA nº 4 467 177 a trait à un article pouvant s'adapter directement au secteur électrique au moyen de fiches ordinaires, lequel article est caractérisé par un double système de chauffage permettant d'atteindre deux niveaux de température: une température élevée, adaptée à l'évaporation de substances insecticides, et une température plus modérée pour l'évaporation d'agents parfumants et désodorisants.

Les dispositifs connus présentent un inconvénient majeur: ils fonctionnent par émission continue des vapeurs assainissantes. Si un tel principe n'est guère gênant pour la diffusion de vapeurs de substance insecticide, son application à la diffusion d'agents parfumants en général provoque rapidement un phénomène d'accoutumance et de saturation olfactive, ce qui se traduit chez l'observateur par une désensibilisation marquée vis-à-vis des agents parfumants communément employés.

L'art antérieur fait aussi état de dispositifs qui pallient à cet inconvénient par une émission séquencée des parfums dans l'atmosphère. C'est ainsi que les brevets européens nºs 104 758 et 123 746 décrivent des dispositifs diffuseurs d'ingrédients parfumants comprenant un boîtier, un élément poreux imprégné d'un parfum et disposé dans ce boîtier entre un élément de chauffage et des orifices supérieurs, l'élément de chauffage étant adapté pour fonctionner de façon pulsée pour permettre d'alterner des périodes d'émission des vapeurs du parfum avec des intervalles pendant lesquels cette émission est interrompue. La durée de ces derniers est réglée de façon à permettre un redressement de la réponse olfactive des individus exposés aux vapeurs parfumantes. Cependant, ces dispositifs de l'art antérieur ne permettent la diffusion que d'un parfum à la fois, l'élément poreux imprégné devant être remplacé si l'on veut varier la nature du parfum diffusé.

La présente invention est basée sur l'observation qu'un renouvellement automatique et séquencé de la nature des agents parfumants actifs dans l'atmosphère pouvait éviter la désensibilisation olfactive de façon encore plus efficace.

L'invention a en effet pour objet un dispositif automatique pour l'activation et la diffusion répétitive et alternée de vapeurs d'agents volatils assainissants, lequel dispositif est caractérisé en ce qu'il comprend:

a. un boîtier comportant en sa base une ouverture et en sa partie supérieure des orifices;

b. un élément de support poreux situé au-dessous desdits orifices et comprenant plusieurs zones, chacune imprégnée d'un agent volatil assainissant de nature distincte, ou plusieurs éléments de support poreux situés en dessous desdits orifices, chacun desdits éléments étant imprégné d'un agent volatil assainissant de nature distincte;

c. un élément de chauffage électrique disposé au-dessous de chacune desdites zones de l'unique élément poreux ou de chacun desdits éléments poreux et dont la disposition est telle que chaque élément de chauffage est actionné séquentiellement et séparément à l'aide d'un commutateur de sorte que, une fois enclenché, il provoque un réchauffement de l'air contenu dans la chambre, formée par les parois du boîtier, et un mouvement de convection de celui-ci à travers l'élément poreux situé juste en-dessus, accompagné d'un appel de l'air environnant à travers l'ouverture à la base du boîtier;

d. un élément séquenceur comprenant un oscillateur apte à contrôler la séquence composée d'une base de temps, et un compteur associé à un système logique relié aux commutateurs des éléments de chauffage de sorte que l'enclenchement de ceux-ci ait lieu de façon séquentielle selon un processus prévoyant, pour chaque élément de chauffage, alternativement une période de réchauffement suivie d'une période de refroidissement.

Le dispositif de l'invention permet donc une diffusion contrôlée dans le temps des vapeurs d'agents volatils assainissant. Dans la mesure où chaque zone de l'unique élément poreux ou chaque élément poreux, s'il y en a plusieurs, peut être imprégné séparément par un agent assainissant distinct, il est possible, grâce à ce dispositif, de faire varier à volonté la nature des parfumé que l'on désire répandre dans un local donné. Le temps de diffusion, déterminé également à volonté par l'utilisateur, pourra être choisi en fonction du volume de ce local ou de la nature du parfum employé, en particulier de sa volatilité. L'alternance enfin des périodes d'activation/diffusion (réchauffement) et des périodes de repos (refroidissement), ces dernières s'accompagnant d'une diminution graduelle de la diffusion, évite le phénomène d'accoutumance auprès de l'utilisateur qui peut ainsi maintenir en éveil constant sa capacité de perception olfactive et percevoir pleinement la nouvelle odeur qui se dégage pendant la nouvelle période d'activation qui suit. Le dispositif de l'invention présente ainsi des avantages certains par rapport aux dispositifs de vaporisation connus.

Le début du cycle d'utilisation peut avoir lieu soit par enclenchement direct de la part de l'utilisateur, par exemple au moyen d'un interrupteur reliant le dispositif au réseau électrique principal, soit par une mise en marche automatique au moyen d'un système par lequel la mise en place de l'élément poreux imprégné d'agent actif volatil enclenche l'alimentation électrique du dispositif et initie le processus séquentiel de diffusion.

Le ou les éléments poreux peuvent être constitués par des supports de nature variée, polymérique, cellulosique, minérale ou autre. Des résines polyoléfiniques, polyamidiques ou acryliques peuvent être employées à cet effet. Toutefois, il est apparu que pour des raisons pratiques et économiques des supports cellulosiques pouvaient parfaitement convenir. C'est ainsi que chaque élément poreux peut être constitué par du carton pressé. Ce matériau est capable, d'une part, de retenir à température ambiante l'agent volatil actif pendant un temps jugé suffisant et, d'autre part, de permettre sa diffusion par chauffage modéré.

Bien entendu, l'élément poreux peut avoir des formes variées. Il peut se présenter par exemple sous forme d'un disque, d'un carré, d'une croix, d'une étoile à plusieurs branches ou d'un trèfle à plusieurs feuilles.

Lorsqu'il y a un seul élément poreux, on veillera à choisir un élément dont la forme permet de placer plusieurs agents volatils actifs différents sur un même élément dans des zones différentes sans qu'ils se mélangent, par exemple un élément en forme de trèfle.

Des formes d'exécution du dispositif de l'invention seront décrites, à titre d'exemples, en se référant aux dessins annexés dans lesquels:

la fig. 1 représente une vue d'un dispositif construit conformément à l'invention;

la fig. 2 représente ce même dispositif en coupe;

la fig. 3 représente une exécution particulière d'un élément poreux comportant quatre zones imprégnées d'agent assainissant;

la fig. 4 est une représentation du schéma électronique proposé pour l'enclenchement séquentiel des éléments de chauffage;

et la fig. 5 représente schématiquement le processus séquentiel de chauffage et refroidissement.

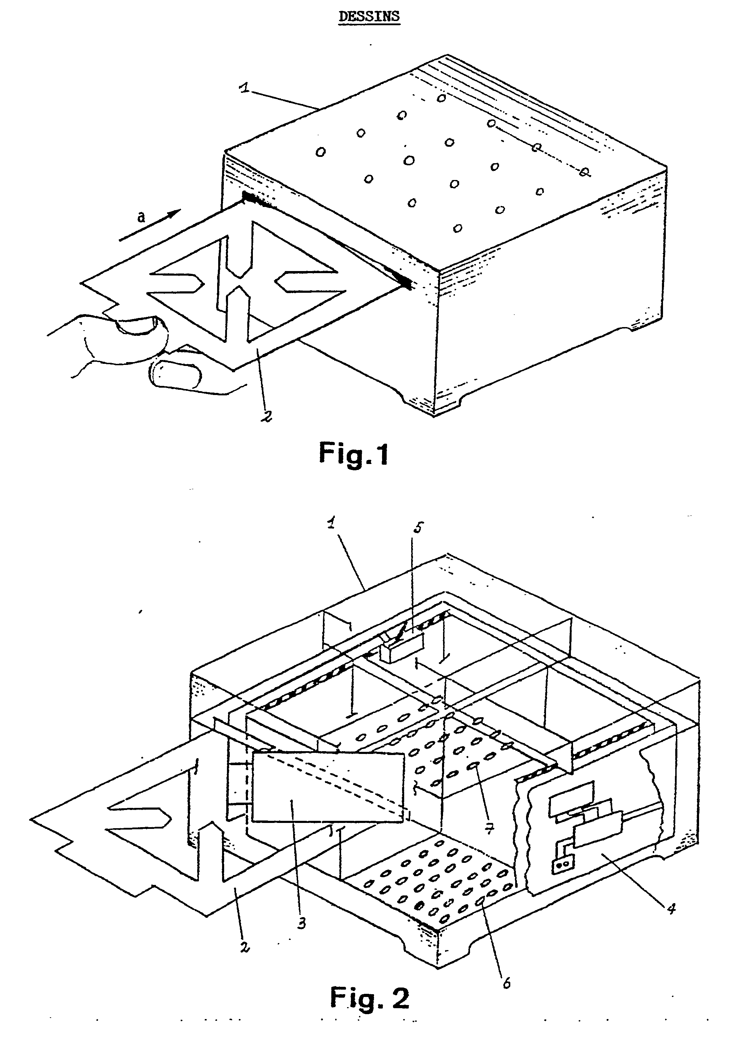

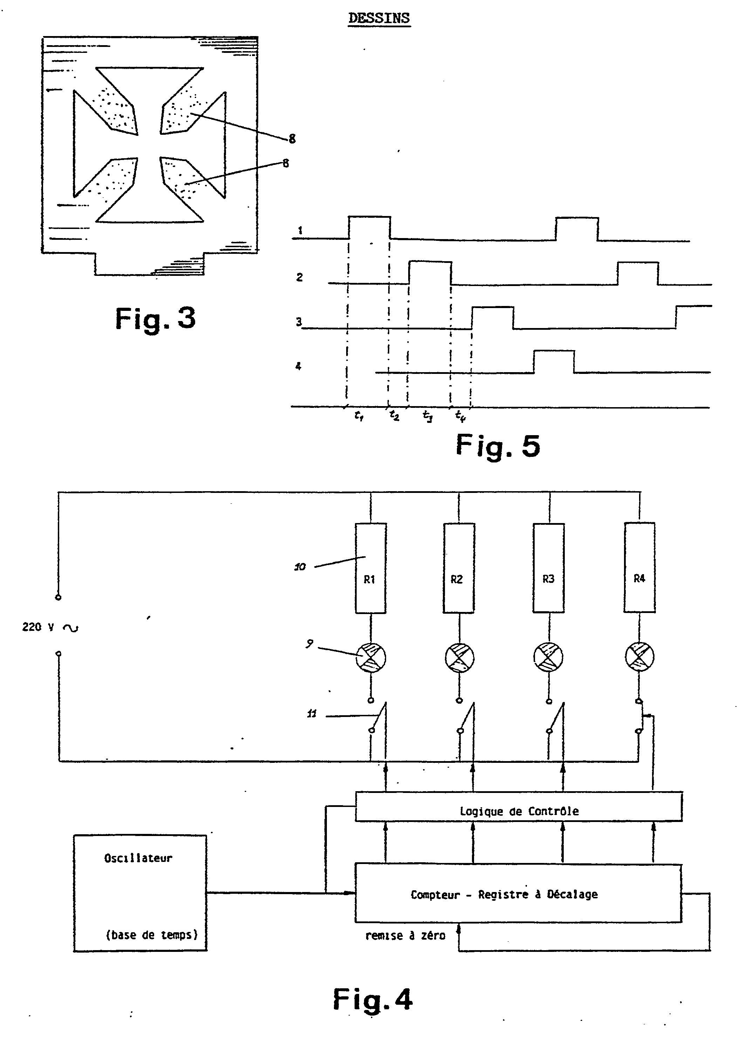

Faisant référence aux différentes figures, la fig. 1 montre un mode d'exécution préférentiel du dispositif de l'invention dans lequel l'élément poreux 2 imprégné d'agent assainissant est introduit dans la fente pratiquée dans l'une des parois du boîtier 1 du dispositif. Une fois introduit complètement, il enclenche l'interrupteur 5 (voir fig. 2), ce qui permet l'alimentation en courant du circuit électronique en plaque 4 et initie le processus de chauffage séquentiel des corps de chauffe (résistances électriques) 3. Ceux-ci sont situés au-dessous de l'élément poreux imprégné de sorte à promouvoir le réchauffement et l'évaporation de la substance volatile assainissante active 8 qui imbibe chaque branche de l'élément poreux (voir fig. 3). La découpe de celui-ci comporte une section plus large sur l'un de ses côtés apte à faciliter la prise par l'utilisateur en vue de son introduction (a) ou de son retrait dans le boîtier.

Lors de l'enclenchement, l'alimentation électrique permet la mise en marche de l'oscillateur (voir fig. 4) qui transmet des impulsions régulières en base temps à un compteur de sorte que pendant un temps $t_1$ (voir fig. 5) le premier des commutateurs 11 reste fermé, ce qui permet l'alimentation de la résistance R4. Un voyant lumineux 9 constitué par une diode peut servir, à titre facultatif, à indiquer l'activation de la résistance. La période de chauffage $t_1$ est suivie par une période de refroidissement $t_2$ au cours de laquelle la résistance R4 reste inactive. La diffusion des vapeurs assainissantes, activée pendant le chauffage par la résistance R4, se ralentit graduellement au cours de cette phase et cesse complètement après quelques instants. Une nouvelle impulsion provenant de l'oscillateur permet la répétition du processus avec fermeture d'un autre commutateur et alimentation de la résistance R3 pendant un temps $t_3$. Ce processus se répète jusqu'à ce que chacune des résistances ait été activée une fois lors d'un cycle complet, et l'ensemble est ainsi prêt pour un nouveau cycle.

Dans le dispositif montré à l'aide des figures indiquées, l'élément poreux qui sert comme support de la substance assainissante volatile est constitué par un élément comportant quatre branches séparées sur chacune desquelles est déposée une substance assainissante distincte. Lors d'un cycle de fonctionnement complet, on obtient donc la diffusion alternée de quatre substances volatiles différentes. Il demeure entendu que, conformément à l'invention, d'autres dispositifs peuvent être construits pour la diffusion d'un nombre variable de substances volatiles; le nombre des éléments de chauffe sera naturellement prévu en fonction de cela.

Un réglage approprié de l'oscillateur à travers une résistance variable permettra d'établir à volonté la valeur des temps de chauffage et de refroidissement. Ces valeurs peuvent être identiques ou différentes. Le choix particulier de la valeur ohmique des résistances (R1 à Rn) permettra également de régler à volonté la température de chauffage, celle-ci étant choisie en fonction de la volatilité, et de la stabilité thermique, de la substance volatile assainissante.

La diffusion de celle-ci est facilitée par la présence d'une ouverture, unique ou sous forme de fentes ou orifices 6 (voir fig. 2), pratiquée à la base du boîtier 1. L'air de la chambre, réchauffé pendant l'activation, diffuse à travers l'élément poreux entraînant avec lui des vapeurs de la substance assainissante qui diffusent à travers les orifices 7 situés dans la partie supérieure du boîtier tandis qu'elle provoque un appel d'air environnant qui pénètre dans la chambre à travers l'ouverture 6.

A titre de substance assainissante, on peut utiliser pour le dispositif de l'invention les substances parfumantes, odorantes ou désodorisantes usuelles ainsi que les substances insecticides, bactéricides, répulsives ou attirant les animaux.

L'agent volatil assainissant peut être choisi parmi une multitude de compositions parfumantes ou d'ingrédients odorants d'utilisation courante en parfumerie. A cet effet, le choix particulier que l'on peut effectuer est tel qu'il est ici superflu de mentionner la nature des différents ingrédients possibles. L'on peut mentionner cependant ceux décrits dans des ouvrages spécialisés, par exemple dans S. Arctander, "Perfume and Flavor Chemicals", Montclair, N.J. (1969). Ces substances sont de préférence employées sous forme de solution aqueuse.

Il demeure entendu que l'utilisation particulière de l'une ou l'autre de ces substances est fonction de l'emploi spécifique et du résultat que l'on se propose d'obtenir. L'utilisateur pourra donc déterminer la nature de la ou des substance(s) désirée(s) et pourra porter son choix parmi la grande variété de substances connues selon son goût et l'emploi souhaité.

**Revendications**

1. Dispositif automatique pour l'activation et la diffusion répétitive et alternée de vapeurs d'agents volatils assainissants, caractérisé en ce qu'il comprend:

   a. un boîtier comportant en sa base une ouverture et en sa partie supérieure des orifices;

   b. un élément de support poreux situé au-dessous desdits orifices et comprenant plusieurs zones, chacune imprégnée d'un agent volatil assainissant de nature distincte, ou plusieurs éléments de support poreux situés au-dessous desdits orifices, chacun desdits éléments étant imprégné d'un agent volatil assainissant de nature distincte;

   c. un élément de chauffage électrique disposé au-dessous de chacune desdites zones de l'unique élément poreux ou de chacun desdits éléments poreux et dont la disposition est telle que chaque élément de chauffage est actionné séquentiellement et séparément à l'aide d'un commutateur de sorte que, une fois enclenché, il provoque un réchauffement de l'air contenu dans la chambre, formée par les parois du boîtier, et un mouvement de convection de celui-ci à travers l'élément poreux situé juste en dessus, accompagné d'un appel de l'air environnant à travers l'ouverture à la base du boîtier;

   d. un élément séquenceur comprenant un oscillateur apte à contrôler la séquence composée d'une base de temps, et un compteur associé à un système logique relié aux commutateurs des éléments de chauffage de sorte que l'enclenchement de ceux-ci ait lieu de façon séquentielle selon un processus prévoyant, pour chaque élément de chauffage, alternativement une période de réchauffement suivie d'une période de refroidissement.

2. Dispositif selon la revendication 1 caractérisé en ce que l'élément poreux est constitué par un support cellulosique.

3. Dispositif selon la revendication 1 caractérisé en ce que l'élément de chauffage est constitué par une ou plusieurs résistances électriques.

4. Dispositif selon la revendication 1 caractérisé en ce qu'il comporte un interrupteur de sorte que sa mise en marche ait lieu par l'enclenchement automatique de celui-ci lors de l'introduction de l'élément poreux dans le boîtier.

**Claims**

1. Automatische Vorrichtung zur wiederholten und alternierenden Aktivierung und Diffusion von flüchtigen, reinigenden Dämpfen eines Stoffes, dadurch gekennzeichnet, daß sie

   a. ein Gehäuse, das an seiner Grundfläche eine Öffnung und an seinem Oberteil Ausgänge hat, b. einen porösen Träger, der unter den obengenannten Ausgängen liegt und mehrere Zonen enthält, wovon jede mit einem verschiedenen, flüchtigen, reinigenden Stoff imprägniert ist, oder mehrere poröse Träger, die unter den obengenannten Ausgängen liegen, wovon jeder der obengenannten Träger mit einem verschiedenen, flüchtigen, reinigenden Stoff imprägniert ist, c. ein elektrisches Heizelement, das unter jeder der obengenannten Zonen des einzigen porösen Trägers, oder unter jedem der obengenannten porösen Träger liegt, und dessen Anordnung so ist, daß jedes der Heizelemente mittels eines Schalters sequentiel und getrennt eingeschaltet wird und daß es, wenn eingeschaltet, eine Erwärmung der Luft in dem Gehäuse und eine Konvektionsbewegung derselben durch den darüberliegenden, porösen Träger, zusammen mit einem Luftzug der umliegenden Luft durch die Öffnung der Gehäusegrundfläche, bewirkt,

   d. ein Sequenzelement, welches aus einem Zeitbasis kontrollfähigen Oszillator und aus einem Zählwerk zusammen mit einem logischen System besteht, und welches mit den Schaltern der Heizelemente so verbunden ist, daß es das sequentielle Einschalten jedes Heizelementes nach einen Prozess, welcher abwechselnd eine Heizphase gefolgt von einer Abkühlphase vorsieht, beinhaltet.

2. Vorrichtung gemäß Anspruch 1, dadurch gekennzeichnet, daß der poröse Träger aus einem Zelluloseträger besteht.

3. Vorrichtung gemäß Anspruch 1, dadurch gekennzeichnet, daß das Heizelement aus einem oder mehreren Widerständen besteht.

4. Vorrichtung gemäß Anspruch 1, dadurch gekennzeichnet, daß sie einen Schalter hat, der sich automatisch eingeschaltet, wenn der poröse Träger in das Gehäuse eingeführt wird.

## Claims

1. Automatic device for the repeated and alternating activation and diffusion of volatile purifying agent vapours, characterised in that it comprises :
    a. a casing having at its base an opening and in its upper part vents;
    b. one porous support element disposed below the said vents and comprising several zones, each zone being impregnated with a distinct volatile purifying agent, or several porous support elements disposed below said vents, each of the said elements being impregnated with a distinct volatile purifying agent;
    c. an electric heating element disposed below each of the said zones of the single porous element or below each of the said porous elements and the positioning of which is such that each heating element is sequentially and separately activated by a switch, such that once activated, it causes the air in the chamber, formed by the walls of the casing, to heat up and creates a convection movement of the air through the porous element located right above it, together with a draught from the surrounding air through the opening in the base of the housing;
    d. a sequence switch element comprising an oscillator capable of controlling the sequence consisting of a time base, and a counter associated with a logic system connected to the switches of the heating elements, so that the engagement of£ said elements occurs in a sequential manner according to a process providing for each heating element, alternatively, a heating period followed by a cooling period.

2. A device according to claim 1, characterized in that the porous element is formed by a cellulose support.

3. A device according to claim 1, characterized in that the heating element is formed by one or more electrical resistors.

4. A device according to claim 1, characterized in that it comprises a switch such that it can be switched on by automatic engagement thereof when the porous element is inserted into the casing.

DESSINS

**Fig.1**

**Fig. 2**

DESSINS

Fig. 3

Fig. 5

Fig. 4